# EUROPEAN PATENT SPECIFICATION

(11) **EP 2 753 369 B1**
(45) Date of publication and mention of the grant of the patent: **09.12.2015**
(21) Application number: 12753750.4
(22) Date of filing: 06.09.2012
(51) Int. Cl.: A61B 17/06, A61L 17/14, A61L 17/00, D06Q 1/10, D06Q 1/14

(54) **FLOCKED SURGICAL SUTURE AND METHODS FOR THE PRODUCTION THEREOF**
BEFLOCKTE CHIRURGISCHE NAHT UND HERSTELLUNGSVERFAHREN DAFÜR
SUTURE CHIRURGICALE FLOQUÉE ET SES PROCÉDÉS DE FABRICATION

(30) Priority: 07.09.2011 EP 11180412
(43) Date of publication of application: 16.07.2014
(73) Proprietor: Aesculap AG, 78532 Tuttlingen/Donau (DE); B. Braun Surgical, S.A., 08191 Rubi (Barcelona) (ES)
(72) Inventor: ODERMATT, Erich, CH-8200 Schaffhausen (CH); PFEIFFER, Ruth Verena, E-08173 Sant Cugat del Vallès (Barcelona) (ES)
(74) Representative: Patentanwälte Ruff, Wilhelm, Beier, Dauster & Partner mbB
(86) International application number: PCT/EP2012/067420
(87) International publication number: WO 2013/034644

(56) References cited:
- WO-A1-2010/127874

## Description

The present invention relates to a flocked surgical suture, methods for producing such a surgical suture and a surgical combination comprising a flocked surgical suture.

Surgical sutures are a commonly used standard for closing or binding together wounds in human or animal tissue, such as skin, muscles, tendons, internal organs, nerves, blood vessels, and the like. In that regard, the surgeon may use a surgical needle with an attached conventional suture so as to peer the tissue alternately on opposing faces of the wound and thus sew the wound closed. After removal of the surgical needle, the ends of the sutures are tied.

In that regard, carefulness has to be applied in order to close the wounds with an optimal force at the wound margins. If the wound margins are sutured too loosely and too irregularly, for example, there is in principle a risk of increased scarring or dehiscence. By contrast, if the wound margins are sutured too strongly, necrotic changes may occur in the surrounding tissue due to a limited circulation of blood in the wound margins.

Further, there is always a certain risk of the wound closure, based on knotting of suture materials, leading to impaired healing and to unsatisfactory cosmetic results.

Furthermore, several knots are typically required in order to achieve a secure wound closure. However, this entails the introduction of a large amount of suture material into the wound zone which may cause undesired foreign-body reactions.

In contrast to the above-described conventional surgical sutures, barbed sutures are not dependent on knotting in order to obtain a secure wound closure.

A barbed suture includes an elongated body that has one or more spaced barbs, which project from the body surface along the body length. The barbs are typically generated by cuts into the surgical suture. Barbed sutures are known, by way of example, from US 3,123,077, EP 1 559 266 B1, EP 1 560 683 B1, and EP 1 556 946 B1.

WO 2010/127874 A1 is directed to knotless or self-fixing surgical suture material which can be pulled through tissue with less trauma (presence of and an additional instrument). This document discloses coated thread with anchoring structures. Anchoring structures are obtained either by incision from the core suture or by addition of a polymeric material. A coating is further applied on the core covered with the anchoring structures in order to facilitate the insertion into the body.

Due to their specific generation, barbs generally contribute to at least a portion-like reduction of suture diameter. However, reducing suture diameter is principally associated with the risk of weakening the mechanical stability of the suture. This does even more apply inasmuch as suture materials are typically drawn, and thus reduced in respect of their diameters, before generating the barbs.

The object of the present invention is therefore to make available a surgical suture which circumvents withdrawals known from sutures of the prior art. In particular, the surgical suture shall improve wound closure, particularly shall allow for a knotless wound closure, while concurrently bearing sufficient mechanical strength in order to contribute to a secure wound closure.

According to a first aspect of the invention, this object is solved by a surgical suture having the features of independent claim 1. Preferred embodiments of the surgical suture are reflected in dependent claims 2 to 16. In accordance with a second and third aspect of the invention, the object underlying the present invention is solved by methods as recited in independent claims 17 and 18. According to a fourth aspect of the invention, the above-mentioned object is solved by a surgical combination having the features of claim 19. The subject matter and wording, respectively of all claims is herewith incorporated into the description by explicit reference.

The surgical suture according to the present invention comprises a suture body and a flock material.

The present invention is based on the surprising finding that flocking technology may be successfully applied to surgical sutures. Flocking sutures yields a new versatile class of surgical sutures exhibiting numerous advantages in relation to conventional sutures:
- Flocking a surgical suture is associated with an enlargement of the suture's surface. This advantageously increases friction forces onto the suture requiring less knotting in order to achieve a secure fixation of the suture. Thus, the input of foreign material into the body of a patient which may be derived from a high number of knots can be avoided.
- Further, increased friction forces of the suture advantageously lead to improved knot-pull strength, thereby contributing to a higher knot security.
- Furthermore, the flock material may endow the suture with self-fixating or self-anchoring properties. In other words, the suture according to the present invention may advantageously be a self-fixating suture, i.e. a suture which does not require any knotting in order to obtain wound closure (knotless suture). In that regard, the flock material may penetrate human and/or animal tissue, thereby achieving an improved fixation of the suture within the tissue, which may be in particular due to higher friction forces.
- Moreover, flocking a suture does not impair or at least not basically impair mechanical characteristics such as tensile strength and knot-pull strength of the suture.
- Besides, depending on the flock material and its characteristics such as diameter, length, titer, or the like, the suture may be equipped with specific properties. Thus, manufacture of sutures for specific areas of application is possible, by choosing a suitable flock material.

In case that the flock material is for example a pharmaceutical drug, medication or drug release may be controlled by characteristics like flock titer.
- Further, a flock suture may advantageously address stitch bleeding which commonly occurs when suturing arteries, especially calcified arteries, thereby threatening patients' safety and increasing operation time. By using a flock suture passing through an arterial wall, the flock material is able to resist the blood pressure and finally hinders the occurrence of stitch bleeding, in particular when flock material is involved being prone to swelling.
- Further, a flock material may be specifically adapted to different implantation sites such as sites demanding for a more lipophilic or hydrophilic structure, an anti-adhesive or adhesive structure, an anti-bacterial structure, and the like.
- Furthermore, the flock material may advantageously contribute to an enlargement of the suture's surface and the suture's volume, thereby making the suture more accessible to additives such as active agents, pharmaceutical drugs, cells, in particular stem cells, and the like.
- Moreover, the flock material may form a retaining structure onto the suture. This makes it particularly easy to finish the suture, by way of example, with additives such as mentioned in the preceding paragraph. In other words, it may also be within the scope of the invention that the surgical suture may be applied as a drug and/or cell release carrier.

The term "suture body" as used according to the present invention may refer to one suture body, i.e. a single suture body, or to a plurality of suture bodies, i.e. to two or more suture bodies. Depending on the indications to be treated, it may be within the scope of the present invention that several suture bodies may be fixed together by a fixation means such as a simple knot or by ultrasound or welding, thereby forming a surgical suture according to the present invention. For example, three or four suture bodies may be fixed together, thereby forming a triangle or quadrupole suture.

The term "flock material" as used according to the present invention defines a material which may be applied on a substrate such as a suture body by means of a flocking technique.

Typically, the suture body is present as an elongate body.

In a preferred embodiment, the surgical suture additionally comprises an adhesive layer, in particular a cured or solidified adhesive layer. However, it may also be conceivable according of the invention that the surgical suture comprises an adhesive layer in a viscous condition.

The adhesive layer may partially or completely layer, in particular coat, ensheath, envelop, encase, surround or wrap, the suture body.

The flock material is preferably attached to the adhesive layer. The adhesive layer advantageously facilitates attachment of the flock material onto the surface, typically onto the exterior surface, of the suture body.

Therefore, in a further preferred embodiment, the flock material is attached to the suture body by means of an adhesive layer.

The flock material may be partly, in particular merely partly, incorporated into the adhesive layer.

Thus, it may be preferred according to the invention that the flock material is not incorporated into the suture body. In other words, it may be preferably that the flock material does not penetrate or invade the suture body.

According to a further embodiment, the flock material is incorporated into the adhesive layer over a length between 0.5 and 95 %, in particular 3 and 50 %, preferably 5 and 20 %, in relation to its total length.

Preferably, the adhesive layer is formed as a non-textile layer, in particular as a coating, sheath, film, gel, particularly hydrogel, or paste. The embodiment as a coating or sheath is especially preferred according to the present invention.

However it may be also conceivable according to the invention that the adhesive layer is present as a textile structure such as a braided, knitted, woven and/or felted structure.

Specifically, the adhesive layer may be present in the form of a fibre or filament or a plurality of fibres or filaments. For example, the adhesive layer may be formed as monofilament, pseudo monofilament and/or multifilament such as intertwined, twisted, textured or braided multifilament. According to the invention it may be especially preferred for the adhesive layer to be formed as a multifilament, in particular as a braid.

According to a specific embodiment, the adhesive layer may be formed as a filament which helically surrounds the suture body. Having regard to useful embodiments for the filament, reference is made to the previous embodiment.

The adhesive layer may have a proportion of from 0.1 to 50 % by weight, in particular from 0.5 to 20 % by weight, preferably from 1 to 10 % by weight, related to the total weight of the surgical suture.

In a further embodiment, the adhesive layer may have a thickness between 1 and 2000 µm, in particular between 3 and 1000 µm, preferably between 5 and 500 µm.

In principle, the adhesive layer may be absorbable, partially absorbable or non-absorbable. In other words, the adhesive may include or be made of an absorbable material, a partially absorbable material or a non-absorbable material. Examples of useful materials are provided in the following.

Typically, the material is a polymer. The polymer may be a synthetic polymer or a biopolymer, i.e. a naturally occurring polymer.

The term "material" or "polymer" as used according to the invention may relate to one, i.e. single, material or polymer or to a mixture of different materials or polymers.

Specifically, the material may be a copolymer.

The term "copolymer" as used according to the present invention, relates to a polymer which is composed of at least two different monomer units. Thus, the term "copolymer" may encompass, by way of example, bipolymers (polymer composed of two different monomer units), terpolymers (polymers composed of three different monomer units) or tetrapolymers (polymers composed of three different monomer units).

Further, a copolymer according to the present invention may be a random copolymer, an alternating copolymer, a segmented or block copolymer, particularly a triblock terpolymer, a graft copolymer or combinations thereof.

The adhesive layer may be distinct from the suture body. The distinct-ness may be based on different chemical and/or physical properties, such as different materials, cross-linking, solubility or swelling characterristics towards physiological, in particular bodily, liquids, thermal stability, melting point, crystallinity, or the like.

According to a more specific embodiment, the adhesive layer may include or be made from a different material, in particular polymer, than the suture body. Alternatively, the adhesive layer may include or be made of the same material as the suture body.

In a further specific embodiment, the adhesive layer may include or be made of a material, typically a polymer, preferably a thermoplastic polymer, having a lower melting temperature than a material, typically a polymer, which is included in the suture body or of which the suture body is made. Thus, it may be within the scope of the present invention to use the same material, in particular polymer, for the adhesive layer and the suture body wherein the material, in particular polymer, for the adhesive layer is a lower melting material, in particular polymer, in comparison to the material, in particular polymer, for the suture body. For example, a polypropylene having a melting temperature of roughly 150 °C may be employed for the adhesive layer, while a polypropylene having a melting temperature of roughly 165 °C may be employed for the suture body. Thus, a specific activation of the adhesive layer may be achieved by applying temperatures which allow for melting the material of the adhesive layer but not the material of the suture body. This advantageously maintains the structural integrity of the suture body.

In a further embodiment, the adhesive layer may include or be made of a material which exhibits adhesive or sticky properties upon adding a liquid. Useful liquids may be water, aqueous solutions, buffer solutions, electrolyte solutions, medicinal or pharmaceutical solutions, aqueous suspensions, organic solvents, and the like, and combinations thereof. In this embodiment, the material is preferably a biopolymer such as a protein and/or polysaccharide. With respect to useful biopolymers reference is made to the following description.

In principle, the adhesive layer may include or be made of any material. In that regard, the adhesive material is not limiting the present invention provided that the material may be activated to exhibit adhesive or sticky properties in order to allow for attaching the flock material onto the suture body.

According to a useful embodiment, the adhesive layer includes or is made of a glue or hot melt. For example, the adhesive layer may be present as a protein and/or polysaccharide glue. Having regard to useful proteins and/or polysaccharides, reference is made to the below description.

According to a more specific embodiment, the adhesive layer may include or be made of a polymer, particularly a non-absorbable or partly absorbable polymer, which is preferably selected from the group consisting of hot melts, in particular based on polyamide, polyimides, polyester, polyolefins, polyethylene, amorphous poly-α-olefin, polybutene-1, ethylene-vinyl acetate, polyurethanes such as thermoplastic polyurethanes or the like, polyvinyl alcohol, polyvinyl acetate, polyvinylchloride, polyvinyl propionate, polyacrylates, cyanoacrylates, polyurethanes, modified vinylacetat copolymer, polyester-elastomer, polyurethane-elastomer, vinylpyrrolidon-vinylacetat copolymer, polyethylene glycol (PEG) based hydrogels, in particular photoactivated polyethylene glycol (PEG) based hydrogels, resins such as synthetic resins, waxes like bee waxes and/or synthetic waxes, and combinations thereof.

Useful resins for the adhesive layer may be hot melts, waxes, resins made of urea, epoxide, polyurethane, melamine, phenol, polyester, polyamide, vinylester, and combinations thereof.

Further, the adhesive layer may include or be made of an absorbable polymer which is preferably selected from the group consisting of polyhydroxyalkanoates, polyglycolide, polylactide, copolymer of glycolic acid and lactic acid (polyglactine), polydioxanone, polyhydroxybutyrate, poly3-hydroxybutyrate, poly-4-hydroxybutyrate, polytrimethylene carbonate, poly-ε-caprolactone, polysaccharides, cellulose derivates such as alkyl-celluloses, methylcellulose, hydroxyalkylcelluloses, hydroxymethylcellulose, hydroxyethylcellulose, hydroxypropylcellulose, carboxyalkylcelluloses, carboxymethylcellulose, copolymers thereof, salts thereof, stereoisomers thereof, and combinations thereof.

In a further embodiment, the adhesive layer includes or is made of a biopolymer, in particular a protein and/or polysaccharide, preferably selected from the group consisting of structural proteins, extracellular proteins, fibrous proteins, globular proteins, oxidized or non-oxidized polysaccharides, amino group bearing polysaccharides, aldehyde group bearing polysaccharides, mucopolysaccharides, salts thereof, stereoisomers thereof, and combinations thereof.

Preferably, the adhesive layer includes or is made of a polymer selected from the group consisting of collagen, gelatine, elastin, reticulin, fibronectin, laminin, fibrin, albumin, starch, amylose, amylopectin, dextran, dextrin, cellulose, chitin, chitosan, hyaluronic acid, dextran sulfate, heparin, heparan sulfate, chondroitin sulfate, dermatan sulfate, salts thereof, stereoisomers thereof, and combinations thereof.

Further, it may be also within the scope of the present invention that the surgical suture is free of a distinctive or proprietary adhesive layer, in particular of an adhesive layer as described in any of the preceding embodiments.

Instead, the flock material may be directly attached to the suture body.

More specifically, it may be preferably that the flock material is directly incorporated into the suture body, in particular into a superficial layer thereof or into substructures such as fibres or filaments thereof.

More preferably, the suture body, in particular a superficial layer thereof, is at least partly, in particular completely, made of a material, in particular polymer, which acts as an adhesive, preferably upon activation. Having regard to a useful material, in particular polymer, reference is made to the materials, in particular polymers, which have been mentioned for the adhesive layer.

According to a further embodiment, the flock material is attached to an adhesive layer formed onto the suture body, particularly as outlined in any of the preceding embodiments, or as an alternative, to a superficial layer of the suture body by means of non-covalent forces such as adhesion forces, Van-der-Waals forces, or the like.

The term "activation" as used according to the present invention includes any process such as melting, heating, soaking, dipping, liquefying, humidifying, radiating, exposing to ultrasonic waves, exposing to chemicals, or the like, transferring an adhesive layer or a superficial layer of the suture body into an adhesive or sticky state allowing for attaching the flock material onto the suture body.

With respect to the flock material, any material may be conceivable as long as the material may be applied onto the suture body by means of a flocking technique.

The flock material may partially or completely, particularly circumferentially, cover the suture body.

In an especially preferred embodiment, the flock material is formed as fibres, in particular as cut or milled fibres.

More specifically, the flock material may be present as a particulate material such as a powder, pulver or granulate, preferably as crushed or pulverised fibres.

The flock material may have a length between 10 µm and 10 mm, in particular between 200 µm and 6 mm, preferably between 300 µm and 4 mm.

Further, the flock material may have a diameter between 5 and 800 µm, in particular 7 and 300 µm, preferably between 10 and 150 µm.

In a further embodiment, the flock material protrudes from the suture body, in particular from an adhesive layer which is formed onto the suture body, over a length between 10 µm and 9.7 mm, in particular between 100 µm and 5.8 mm, preferably between 200 µm and 3.9 mm.

Furthermore, the flock material may have a linear mass density between 1 and 1000 dtex, in particular between 1.5 and 500 dtex, preferably between 2 and 200 dtex. The dimension "dtex" (deci tex) means a linear mass density of 1 g per 10 000 m length of the flock material.

Typically, the flock material protrudes from the surface of an adhesive layer or from a superficial layer of the suture body which is able to act as an adhesive upon activation. Preferably, the flock material may protrude perpendicular or essentially perpendicular from the adhesive layer or superficial layer of the suture body. The term "essentially perpendicular" as used herein may include aberrations up to 5 degrees from the right angle. However, it may be also conceivable that the flock material protrudes in an acute angle from the adhesive layer or superficial layer of the suture body. An acute angle according to the present invention preferably relates to an angle which is more than 0 degrees but less than 85 degrees.

Moreover, the flock material may comprise a two-dimensional, in particular pattern-like, disposition onto the suture body, in particular onto an adhesive layer formed onto the suture body. The disposition is preferably selected from the group consisting of a linear disposition, a staggered disposition, a helical or spiral disposition, a meander-like disposition, a serpentine-like disposition, a sinus-like disposition, an annular disposition, and combinations thereof. A random or irregular disposition may also be conceivable according to the invention.

For example, a helical or a spiral disposition may be realized by means of a filament, typically a monofilament, which is helically or spirally wound onto the suture body and which may act as an adhesive layer for the flock material. Alternatively, a helical pattern may be realized using a braided suture whose filaments are at least partially made of a thermoplastic polymer having a lower melting temperature than the polymers of other filaments of the suture. Thus, the thermoplastic filaments may be activated as adhesive filaments which advantageously may lead to a helix-shaped flock pattern.

Further, the flock material may be present onto the suture body in a unidirectional or multidirectional, in particular bidirectional, disposition. A unidirectional disposition as used herein means a disposition where the flock material is oriented in one direction, while a bidirectional disposition relates to a disposition where the flock material is oriented in two, preferably two opposing, directions.

According to a further embodiment, the flock material comprises a section-like or repeating disposition, preferably an annular disposition, onto the suture body, in particular onto an adhesive layer being formed onto the suture body. Annular dispositions of the flock material may be present at one or both ends and/or in the middle of the suture body. In that regard, annular dispositions may advantageously act as control, marker, stopper or reference elements for the surgeon. For example, annular dispositions being spaced from each other by about 5 mm provides the surgeon with clear information of the final tension he has applied during suturing and incision.

Thus, it may be further within the scope of the present invention that the surgical suture comprises at least one, in particular one, terminal stopper element, i.e. a stopper element being disposed at one end of the surgical suture. The stopper element is preferably provided by the flock material, in particular by annularly disposed flock material.

In principle, the flock material may be absorbable, partially absorbable or non-absorbable. In other words, the flock material may include or be made of an absorbable, a partially absorbable or non-absorbable material.

Further, the flock material may include or be made of a material which is swellable upon contact with water or aqueous media such as aqueous solutions, aqueous suspensions, aqueous buffers and/or bodily liquids such as blood. With respect to useful materials, reference is made to the following description.

The flock material may include or be made of a polymer, particularly a synthetic polymer and/or a biopolymer. Moreover, the flock material may include or be made of a copolymer. In particular, the flock material may include or be made of a terpolymer, for example a triblock terpolymer.

More specifically, the flock material may include or be made of a synthetic polymer which is preferably selected from the group consisting of polyolefins, polyvinyl alcohols, polyamides, polyimides, polyesters, polyurethanes, in particular thermoplastic polyurethanes, polyhydroxyalkanoates, copolymers thereof, and combinations thereof.

In a further embodiment, the flock material may include or be made of a biopolymer or a related polymer thereof which is preferably selected from the group consisting of structural proteins, extracellular proteins, fibrous proteins, globular proteins, enzymes, antibodies, blood clotting factors, oxidized or non-oxidized polysaccharides, amino group bearing polysaccharides, aldehyde group bearing polysaccharides, mucopolysaccharides, salts thereof, stereoisomers thereof, and combinations thereof.

According to a more specific embodiment, the flock material may include or be made of a material which is selected from the group consisting of polyethylene, low-density polyethylene, high-density polyethylene, high-molecular-weight polyethylene, ultra-high-molecular-weight polyethylene, polypropylene, polyethylene terephthalate, polypropylene terephthalate, polybutylene terephthalate, polyamide 6, polyamide 6-6, polyamide 6-12, poylamide 12, rayon, silk, in particular spider silk, polytetrafluorethylene, polyvinylidene dichloride, polyvinylidene difluoride, polytetrafluorpropylene, polyhexafluorpropylene, polyvinyl alcohol, polyglycolide, polylactide, polydioxanone, polyhydroxybutyrate, poly-3-hydroxybutyrate, poly-4-hydroxybutyrate, polytrimethylene carbonate, poly-ε-caprolactone, collagen, gelatine, elastin, reticulin, fibronectin, laminin, fibrin, fibrinogen, albumin, starch, amylose, amylopectin, dextran, dextrin, cellulose, cellulose derivatives such as alkylcellulose, methylcellulose, hydroxyalkylcellulose, hydroxymethylcellulose, hydroxyethylcellulose, hydroxypropylcellulose, carboxyalkylcellulose, carboxymethylcellulose, chitin, chitosan, hyaluronic acid, dextran sulfate, heparin, heparan sulfate, chondroitin sulfate, dermatan sulfate, copolymers thereof, salts thereof, stereoisomers thereof, and combinations thereof.

The term "stereosiomers" as used according to the present invention may include, by way of example, enantiomers, diastereomers and/or racemates such as L- and/or D-configured stereoisomers like L- and/or D-lactide.

A preferred copolymer for the flock material is made of glycolide and lactide, in particular in a weight ratio from 9 : 1 to 1 : 9, in particular 7 : 3 to 3 : 7.

A preferred terpolymer for the flock material may be a terpolymer, in particular a triblock terpolymer, made of glycolide, trimethylene carbonate and ε-caprolactone. Such a terpolymer is commercially available under the name Monosyn^{®}.

In a further preferred embodiment, the flock material may be a physiologically active material. The term "physiologically active material" as used herein preferably means a material that is able to exercise a beneficial effect in vivo under a medical point of view, preferably in respect of an optimized, in particular accelerated, wound healing.

In a more specific embodiment, the flock material may include or be made of an active agent, in particular a cosmetic, biological, pharmaceutical and/or medicinal agent.

Useful active agents are preferably selected from the group consisting of antimicrobial agents, in particular antibiotics, disinfectants, oncological agents, wound-healing agents, anti-scarring agents, inflammation inhibitors, pain-killing agents, growth factors, cellular differentiating factors, cellular adhesion factors, cellular recruiting factors, cell receptors, cell binding factors, cytokines, peptides, structural or extracellular proteins such as collagen, fibrin, fibrinogen, polysaccharides such as hyaluronic acid, oligonucleotides, polynucleotides, DNA, RNA, salts thereof, stereosiomers thereof, and combinations thereof.

In a more specific embodiment, the active agent is selected from the group consisting of fibroblast growth factor (FGF), transforming growth factor (TGF), platelet derived growth factor (PDGF), epidermal growth factor (EGF), granulocyte-macrophage colony stimulation factor (GMCSF), vascular endothelial growth factor (VEGF), insulin-like growth factor (IGF), hepatocyte growth factor (HGF), interleukin-1B (IL-1 B), interleukin-8 (IL-8), nerve growth factor (NGF), bone morphogenetic proteins (BMPs) such as BMP-1 and/or BMP-2, and combinations thereof.

Suitable antimicrobial agents may be selected from the group consisting of biguanide, polyhexamethylene biguanide (PHMB), triclosan, gentamycin, copper, zinc, silver, gold, salts thereof, stereoisomers thereof, and combinations thereof.

A preferred anti-scarring agent may be an angiotensin I enzyme inhibitor such as captopril.

The flock material may have a proportion between 0.01 and 50 % by weight, in particular between 0.5 and 20 % by weight, preferably between 1 and 10 % by weight, related to the total weight of the surgical suture.

In principle, the flock material may represent one type of flock material or different types of flock material. Different types of flock material may be different in respect of material, length, height, diameter, density, dispositions or the like. In that regard, reference is explicitly made to present description.

A preferred embodiment providing different types of flock material concerns a surgical suture comprising a suture body comprising unidirectionally orientated flock material and a terminally positioned stopper element onto the suture body, wherein the stopper element is formed from a further flock material, in particular an annularly disposed flock material. The height and length, respectively of the further, in particular annularly disposed, flock material is preferably higher and longer, respectively than the height and length, respectively of the unidirectionally orientated flock material.

The suture body may comprise at least one fibre, in particular one fibre or a plurality of fibres.

More specifically, the suture body may be a monofilament, a pseudo monofilament or a multifilament, in particular textured, intertwined, twisted or braided multifilament. In other words, the suture body may be formed as a monofile, pseudo monofile or multifile thread or yarn, in particular as a textured, intertwined, twisted or braided thread or yarn.

According to a further specific embodiment, the suture body may be formed as a braid, in particular as a circular or rounded braid, preferably comprising a core.

In principle, the suture body may be absorbable, partially absorbable or non-absorbable. In other words, the suture body may include or be made of an absorbable, a partially absorbable or non-absorbable material.

Further, the suture body may include or be made of a polymer, particularly a synthetic polymer and/or a biopolymer. Moreover, the suture body may include or be made of a copolymer. Besides, the suture body may include or be made of a terpolymer, in particular a triblock terpolymer.

More specifically, the suture body may include or be made of a synthetic polymer which is preferably selected from the group consisting of polyolefins, polyvinyl alcohols, polyamides, polyimides, polyesters, polyurethanes, in particular thermoplastic polyurethanes, polyhydroxyalkanoates, copolymers thereof, and combinations thereof.

In a further embodiment, the suture body may include or be made of a biopolymer or a related polymer thereof which is preferably selected from the group consisting of structural proteins, extracellular proteins, fibrous proteins, globular proteins, oxidized or non-oxidized polysaccharides, amino group bearing polysaccharides, aldehyde group bearing polysaccharides, mucopolysaccharides, salts thereof, stereoisomers thereof, and combinations thereof.

In a more specific embodiment, the suture body may include or be made of a material which is selected from the group consisting of polyethylene, low-density polyethylene, high-density polyethylene, high-molecular-weight polyethylene, ultra-high-molecular-weight polyethylene, polypropylene, polyethylene terephthalate, polypropylene terephthalate, polybutylene terephthalate, polyamide 6, polyamide 6-6, polyamide 6-12, polyamide 12, rayon, silk, in particular spider silk, polytetrafluorethylene, polyvinylidene dichloride, polyvinylidene difluoride, polytetrafluorpropylene, polyhexafluorpropylene, polyvinyl alcohol, polyglycolide, polylactide, polydioxanone, polyhydroxybutyrate, poly-3-hydroxybutyrate, poly4-hydroxybutyrate, polytrimethylene carbonate, poly-ε-caprolactone, collagen, gelatine, elastin, reticulin, fibronectin, laminin, fibrin, albumin, starch, amylose, amylopectin, dextran, dextrin, cellulose, cellulose derivatives such as methylcellulose, hydroxymethylcellulose, hydroxyethylcellulose, hydroxypropylcellulose, carboxymethylcellulose, chitin, chitosan, hyaluronic acid, dextran sulfate, heparin, heparan sulfate, chondroitin sulfate, dermatan sulfate, copolymers thereof, salts thereof, stereoisomers thereof, and combinations thereof.

A preferred homopolymer for the suture body is polyethylene or polypropylene. Unflocked polyethylene or polypropylene sutures suffer from the principle withdrawal that they are difficult to knot. Conversely, flocking a suture body made of polyethylene or polypropylene advantageously allows for a reduction of knot throws. In that regard, absorbable and/or non-absorbable flock material may be employed.

A preferred copolymer for the suture body is made of glycolide and lactide, in particular in a weight ratio from 9 : 1 to 1 : 9, in particular 7 : 3 to 3 : 7.

A preferred terpolymer for the suture body may be a terpolymer, in particular a triblock terpolymer, made of glycolide, trimethylene carbonate and ε-caprolactone.

According to a further preferred embodiment, the surgical suture comprises a suture body, and adhesive layer and a flock material, wherein the suture body is made of polypropylene, the adhesive layer is based on polyurethane and the flock material is formed as polyglycolid fibres. The polyglycolid fibres may have a length of about 0.3 mm.

In a further suitable embodiment, the surgical suture comprises a suture body, an adhesive layer and a flock material, wherein the suture body is made of polypropylene, the adhesive layer is based on polyurethane and the flock material is formed as powdered polypropylene.

According to a further useful embodiment, the surgical suture comprises a suture body, an adhesive layer and a flock material, wherein the suture body is made of polypropylene, the adhesive layer is made of poly-4-hydroxybutyrate that may be activated by heat and the flock material is formed as powdered polyglycolide.

According to another preferred embodiment, the surgical suture comprises a suture body, an adhesive layer and a flock material, wherein the suture body is made of polyglycolide or a copolymer made of glycolide and lactide, the adhesive layer is made of poly-4-hydroxybutyrate that may be activated by heat and the flock material is formed as fibres made of polyglycolide. Polyglycolide fibres may, by way of example, have a length of about 1 mm.

The surgical suture in whole may be absorbable, partially absorbable or non-absorbable.

In principle, the suture body, the flock material and an optionally present adhesive layer can include or be made of the same material or of different materials. With respect to useful materials, in particular polymers, reference is made in its entirety to the previous description.

It may be preferred within the scope of the present invention that the surgical suture is a partially absorbable or even a completely absorbable suture. This may be advantageous inasmuch as an in vivo absorption of a material is normally accompanied by tissue ingrowth and/or remodelling processes which promote wound healing. For example, it may be preferably that the suture body is made of a non-absorbable material, whereas the flock material, in particular along with an optionally present adhesive layer, is made of an absorbable material. Alternatively, the suture body and flock material, in particular along with an optionally present adhesive layer, may be made of an absorbable material. With respect to a useful material, reference is made in its entirety to the complete description.

Preferred embodiments involving a braided suture body are detailed in the following.

According to a preferred embodiment, the suture body is a braid made of polyglycolic filaments or made of filaments made of a copolymer of glycolic acid and lactic acid. In this embodiment, the adhesive layer is preferably a coating made of copolymer of glycolic acid and lactic acid. According to an alternative embodiment, the suture body is a braid made of a non-absorbable polyester such as polyethylene terephthalate, while the adhesive layer is preferably made of a hot melt adhesive.

According to a further alternative embodiment, the suture body is a braid made of silk, while the adhesive layer is preferably made of paraffin.

According to a further preferred embodiment, the surgical suture has a core-sheath structure.

Preferably, the core is provided by the suture body, while the sheath is provided by a material which preferably acts as an adhesive upon activation.

More preferably, the core is provided by the suture body, while the sheath is provided by an adhesive layer, in particular by an adhesive layer as described in any of the preceding embodiments.

Specifically, the core may be made of a non-absorbable material, while the sheath may be made of an absorbable material. This embodiment advantageously allows for a surgical suture having a sufficient high mechanical strength at the beginning and, nonetheless a sufficient basic mechanical strength after absorption of the sheath for the remaining residence time within a body's patient.

Preferably, the surgical suture according to the present invention comprises a core-sheath structure wherein the core, sheath and flock material each are made of an absorbable material.

According to an especially preferred embodiment, the core is made of a terpolymer, in particular triblock terpolymer, made of glycolide, trimethylene carbonate and ε-caprolactone and the sheath is made of polydioxanone.

In an alternative embodiment, the core is made of a terpolymer, in particular triblock terpolymer, made of glycolide, trimethylene carbonate and ε-caprolactone and the sheath is made of polyhydroxybutyrate, in particular poly-4-hydroxybutyrate.

In a further preferred embodiment, the core is made of polydioxanone and the sheath is made of DL-lactide. Preferably, the sheath has a proportion of D-lactide of 70 weight-% and a proportion of L-lactide of 30 weight-%, each related to the total weight of the sheath.

According to a further embodiment, the core is made of a terpolymer, in particular a triblock terpolymer, made of glycolide, trimethylene carbonate and ε-caprolactone, while the sheath is made of DL-lactide. Preferably, the sheath has a proportion of D-lactide of 70 weight-% and a proportion of L-lactide of 30 weight-%, each related to the total weight of the sheath.

In a further preferred embodiment, the core is made of polydioxanone, while the sheath is made of polyhydroxybutyrate, in particular poly-4-hydroxybutyrate.

In a more specific embodiment, the core and the flock material each are made of a terpolymer, in particular triblock terpolymer, preferably made of glycolide, trimethylene carbonate and ε-caprolactone, while the sheath is made of polydioxanone. In this embodiment, it is further preferred that the flock material is formed as fibres, for example having a length of 0.2 mm.

In a further specific embodiment, the core and the flock material each are made of a terpolymer, in particular triblock terpolymer, preferably made of glycolide, trimethylene carbonate and ε-caprolactone, whereas the sheath is made of poly-3-hydroxybutyrate, poly-4-hydroxybutyrate or copolymers thereof. In this embodiment, it is preferred that the flock material is present as a powder.

According to a further specific embodiment, the core and the flock material each are made of polydioxanone, while the sheath is made of poly-4-hydroxybutyrate. In this embodiment, it is especially preferred that the flock material is configured as fibres, which, for example, may have a length of 0.3 mm.

In a further specific embodiment, the core and flock material each are made of polydioxanone, while the sheath is made of lactide, in particular DL-lactide (amorphous). In this embodiment, it is preferable that the flock material is present as a powder.

The following two specific embodiments relate to a surgical suture according to the present invention which is not dependent on an additional adhesive layer in order to attach the flock material onto the suture body.

According to a further embodiment, the suture body may be made of polydioxanone, while the flock material is made of poly-4-hydroxybutyrate. According to a further specific embodiment, the suture body comprises a core-sheath structure wherein the core and sheath are both made of polydioxanone, wherein the polydioxanone for the sheath has a lower melting point than the polydioxanone for the core. As regards the embodiments described in this paragraph, it is preferably that the flock material is configured as fibres, for example having a length of 0.2 mm. According to a further embodiment, the suture body is made of polypropylene, while the flock material is made of polydioxanone. In this embodiment, it is preferably that the flock material is formed as fibres, for instance having a length 0.4 mm.

Further, the suture body and/or the flock material may have a circular and/or non-circular cross-section. Conceivable non-circular cross-sections may be selected from the group consisting of oval cross-section, ellipsoid cross-section, polygonal cross-section such as triangular cross-section, square cross-section, rectangular cross-section, diamond cross-section, pentagonal cross-section, hexagonal cross-section, star-like cross-section, and combinations thereof.

As already mentioned, the surgical suture, in particular the suture body, the flock material and/or an optionally present adhesive layer, may be finished or equipped with additives such as active agents, cells, particularly stem cells, dyes, softening agents, radiopaque agents, marking agents, nanoparticles, or the like. With respect to useful active agents reference is explicitly made to the previous description, in particular to the active agents mentioned in respect of the flock material.

Useful cells may be selected from the group consisting of fibroblasts, platelets, in particular platelet rich plasma fractions, chondrocytes, osteocytes, osteoblasts, adipocytes, miocytes, neurones, astrocytes, oligodentrocytes, hepatocytes, pancreatic cells, progenitor cells thereof, stem cells thereof, in particular mesenchymal stem cells thereof, and combinations thereof.

In general, stem cells, in particular mesenchymal stem cells, may be especially preferred.

In principle, the cells may be autologous, allogenic and/or xenogenic cells. However, in order to minimize the risk of immunological responses, it is especially preferred to use cells of autologous origin.

Thus, as already mentioned, the surgical suture according to the present invention may be employed as a drug and/or cell delivery system or cell carrier.

The flock material according to the present invention may - as also already mentioned - advantageously act as a self-anchoring or self-fixating material, thereby delivering a surgical suture which is not dependent on knotting techniques. In other words, the surgical suture according to the present invention is preferably a self-anchoring, self-fixating or knotless surgical suture.

Further, the surgical suture according to the present invention may generally be used for closing and binding together wounds in human and/or animal tissue, such as skin, muscles, tendons, internal organs, nerves, blood vessels, ligaments and the like. Thus, the surgical suture is principally useful for closing and binding together wounds in soft tissues like connective tissues and in hard tissues like bone or cartilage.

Preferably, the surgical suture may be applied in the field of endoscopic surgery, in particular laparoscopic surgery, microsurgery and/or plastic surgery. In the field of plastic surgery, the surgical suture may be specifically employed for eyebrow face lifting and/or skin lining. Further, due to the typically voluminous enlargement of the suture's surface caused by the flock material, the suture according to the present invention may be also beneficially used as a filling material or filling means, in particular as a cosmetic and/or surgical filling material and means, respectively such as for wrinkles in the skin and/or for aneurysms, in particular brain aneurysms.

The use of the surgical suture for filling aneurysms, in particular brain aneurysms, is especially preferred. In that case it is further preferred that the suture body and the flock material each include polyamide or each are made of polyamide. With regard to suitable polyamides, reference is made in its entirety to the present description. In contrast to conventional approaches to treat aneurysms (such as clips, coiled wires, adhesives, and the like), a surgical suture according to the present invention significantly lowers the risk of aneurysm damage. Insertion of the suture may be facilitated by means of appropriate thin catheters or pushers.

A second aspect of the present invention relates to a method of manufacturing or producing a surgical suture, in particular in accordance with the present invention. The method comprises the steps of
a) layering a suture body with an adhesive,
b) flocking the adhesive-layered suture body with a flock material while the adhesive is still active, in particular wet, or upon activation of the adhesive.

The suture body may be partially or completely layered with the adhesive.

Preferably, step a) is achieved by means of extruding, injecting, casting, immersing, dipping, spraying, brushing, painting and/or rolling techniques.

Preference of the afore-enumerated application techniques depends on the condition of the adhesive. For example, if the adhesive is made of a meltable material, the adhesive may be applied onto the suture body by means of extrusion, in particular by means of sheath extrusion.

In case that the suture body is also made of a meltable material, step a) may be achieved by coextrusion of the suture body and the adhesive.

If the adhesive is present as an aqueous liquid, in particular aqueous solution, it may be preferably to layer the suture body with the adhesive by means of spraying or, as an alternative, by immersing or dipping the suture body into the adhesive containing liquid.

In case that the adhesive is present in a paste-like or gel-like, particularly hydrogel-like, condition, it may be more convenient to apply the adhesive onto the suture body by means of brushing, painting, rolling, or the like.

The phrase "while the adhesive is still active" relates according to the present invention to any condition of the adhesive under that the adhesive is able to attach the flock material onto the suture body during a flocking process.

Preferably, the adhesive is activated by means of heating, irradiating such as ultraviolet or infrared irradiating, exposing to ultrasonic waves, liquefying such as melting, humidifying wetting, soaking, swelling, partial dissolving, partial dispersing, exposing to chemicals, and the like.

Flocking is normally understood as a process of depositing flock material being preferably configured as milled, crushed or pulverized fibres, i.e. small fibre particles (called flock), onto a surface of a substrate.

The flocking may be achieved by means of spreading, spraying, blowing, vibration and/or electrostatic techniques.

According to the present invention, it is especially preferred to flock the adhesive-layered suture body by means of electrostatic flocking technology.

Electrostatic flocking relates to a flocking technique which is aided by a high-voltage electric field. Typically, the surface of a substrate which is to be flocked is coated with an adhesive. Then, flock material is applied electrostatically onto the substrate while the adhesive is still wet. Electrostatic flocking may be performed in a specific flocking apparatus. Typically, the flocking apparatus is given a negative charge, while the substrate is earthed. By doing so, the flock material normally flies vertically onto the substrate attaching to the previously applied adhesive.

If electrostatic flocking is employed to manufacture the suture according to the invention, the flock material, preferably formed as fibres, may be straightened in the electrostatic field and in particular may be fixed in an orientated manner onto the suture body. Due to a high acceleration of the flock material in the electrostatic field, a good incorporation of the flock material into the adhesive layer, in particular good penetration or invasion of the adhesive layer with the flock material, may be achieved.

In order to perform step b), the suture body is preferably applied as an electrode, in particular as an earthed electrode, along with a counterelectrode, in particular in the form of a high-voltage electrode. More preferably, the suture body is applied as a cathode and the counterelectrode is preferably applied as an anode.

The electrodes may have a mutual distance from 2 to 200 cm in particular from 5 to 100 cm, preferably from 7 to 50 cm.

In a further embodiment, a voltage from 5 to 120 kV (kilo Volt), in particular from 10 to 100 kV (kilo Volt), preferably from 20 to 80 kV (kilo Volt), may be applied.

Typically, after flocking the adhesive-layered suture body, i.e. after performing step b), a drying step is carried out.

Advantageously, after flocking the adhesive-layered suture body and prior to a drying step, the flock material may be tilted back or over, in particular pressed onto the suture body, and/or brushed so as to accomplish specific and desired effects. In particular, the flock material may be evenly melted. Preferably the flock material may be partially melted, thereby yielding flock material, in particular flock fibres, having a head at the free, in particular protruding, ends of the flock material. In other words, according to the present invention it may be advantageously that the flock material, preferably in the form of fibres, comprises heads at the free ends, i.e. at the ends which are not attached to the suture body.

In a further useful embodiment, after performing the flocking step b) the flock material onto the suture body may be subjected to thermal fixation. This in particular useful in respect of generating specifically orientated flock material such as bidirectionally orientated flock material onto the suture body.

With respect to further features and advantages of the method according to the second aspect of the invention, in particular in respect of the surgical suture, suture body, adhesive or adhesive layer and/or flock material, reference is made in its entirety to the previous description.

A third aspect of the present invention relates to a method of manufacturing or producing a surgical suture, in particular in accordance with the present invention, comprising the steps of
a) providing a suture body which is at least partly made, in particular completely made, of a material which acts as an adhesive upon activation,
b) flocking the suture body with a flock material upon activation of the suture body's material.

With respect to further features and advantages of the method according to the third aspect of the present invention, in particular in respect of embodiments for step a), step b), process characteristics, process parameters, suture, suture body, material which may act as an adhesive upon activation and/or flock material, reference is made in its entirety to the previous description, in particular to the description of the method according to the second aspect of the present invention.

A fourth aspect of the present invention relates to a surgical combination, particularly a surgical kit, comprising a surgical suture, in particular in accordance with the present invention, and at least one surgical needle.

Preferably, the surgical kit comprises one surgical needle or two surgical needles.

The at least one surgical needle typically comprises a grip portion, a rectilinear portion typically extending from the grip portion and a head portion. Depending on the needle type, the head portion may be configured as an arcuate, spiral or helical portion.

Advantageously, the at least one surgical needle comprises means for attaching the surgical suture to the at least one surgical needle. Such attaching means may be configured in the grip portion, in particular in an area where the rectilinear portion emerges from the grip portion, and/or in the head portion. Useful attaching means may be, by way of example, configured as an eye or hole. Besides, the surgical suture may be attached to the at least one surgical needle by means of gluing, welding such as ultrasound welding, stitching, clamping, and the like.

With respect to further features and advantages, in particular in respect of the surgical suture, reference is made in its entirety to the previous description.

Further features and advantages of the invention will become clear from the following description of preferred embodiments in form of figures, figure descriptions and examples in conjunction with the subject matter of the dependent claims. The individual features can be realized either singularly or severally in combination in one embodiment of the invention. The preferred embodiments merely serve for illustration and better understanding of the invention and are not to be understood as in any way limiting the invention.

The figures schematically show:
- Fig. 1:: A surgical suture according to the present invention,
- Fig. 2:: a further embodiment of a surgical suture according to the present invention,
- Fig. 3:: a further embodiment of a surgical suture according to the present invention,
- Fig. 4:: a further embodiment of a surgical suture according to the present invention,
- Fig. 5:: a further embodiment of a surgical suture according to the present invention,
- Fig. 6:: a further embodiment of a surgical suture according to the present invention,
- Fig. 7:: a further embodiment of a surgical suture according to the present invention,
- Fig. 8:: a further embodiment of a surgical suture according to the present invention,
- Fig. 9:: a picture of a surgical suture according to the present invention and
- Fig. 10:: a further picture of a surgical suture according to the present invention.

### Figure description

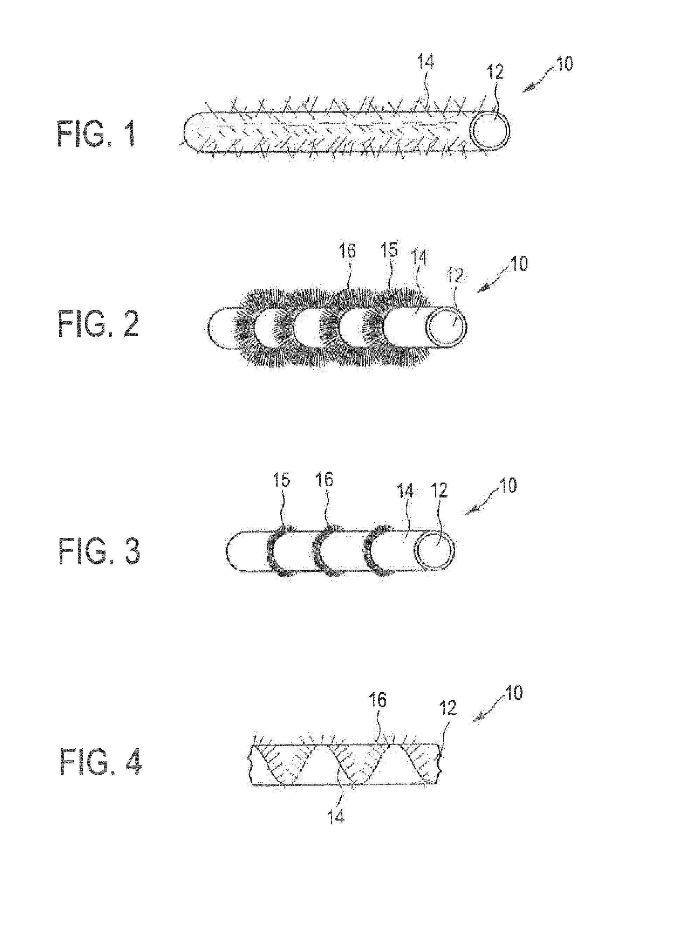

Figure 1 schematically shows a surgical suture 10 according to the present invention. The suture 10 comprises a suture body 12, an adhesive layer 14 and a flock material 16. Typically, the adhesive layer 14 completely enwraps the suture body 12. The flock material 16 is attached to the suture body 12 via the adhesive layer 14, thereby typically protruding from the adhesive layer 14. The suture body 12 is densely covered with the flock material 16.

Instead of an adhesive layer 14 which is distinct from the suture body 12, it may be also conceivable that a superficial layer of the suture body 12 acts as an adhesive layer (not depicted).

Figure 2 schematically shows a further embodiment of a surgical suture 10 according to the present invention. The suture 10 comprises a suture body 12, an adhesive layer 14 encasing the suture body 12 and a flock material 16 in the form of fibres. The fibres 16 are arranged in the form of annular sections 15 on the surface of the encased suture body 12.

Figure 3 schematically shows a surgical suture 10 according to the present invention. The suture 10 comprises a suture body 12, an adhesive layer 14 enveloping the suture body 12 and a flock material 16 in the form of a powder, wherein the powder 16 is arranged in annular sections 15 on the surface of the enveloped suture body 12. The annular sections 15 may advantageously act as fixation means and/or marker means.

Figure 4 schematically shows a surgical suture 10 according to the present invention. The suture 10 comprises a suture body 12, an adhesive layer 14 and a flock material 16. The adhesive layer 14 is present in the form of a meltable filament which helically surrounds the surface of the suture body 12. The flock material 16 being present in the form of fibres sticks to the filament 14.

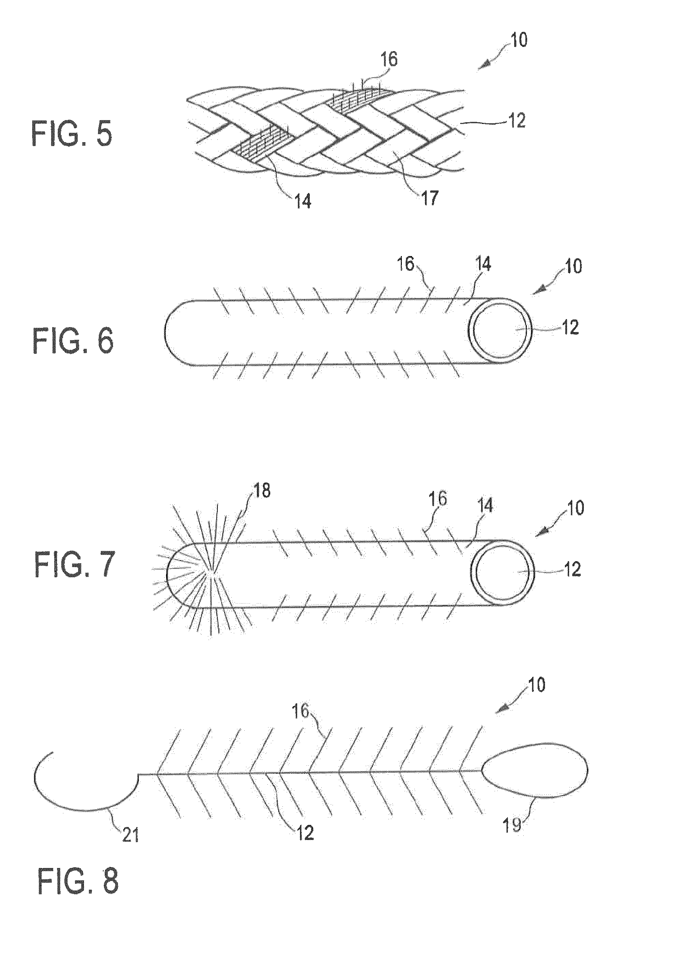

Figure 5 schematically displays a braided surgical suture 10 according to the present invention comprising a suture body 12 being composed of filaments 14 having a low melting temperature and filaments 17 having a high melting temperature. Due to their comparatively low melting temperature, the filaments 14 are advantageously able to act as an adhesive layer within the spirit of the present invention. Thus, a flock material 16 may be attached to the filaments 14, thereby leading to specific flock patterns depending on the course of the filaments 14 within the braided suture 10.

Figure 6 schematically displays a surgical suture 10 comprising a suture body 12, an adhesive layer 14 and a flock material 16. The flock material 16 is bidirectionally disposed on the surface of the suture body 12 and the adhesive layer 14, respectively. For example, a bidirectional disposition of the flock material 16 may be obtained by thermal fixation following the flocking of the adhesive layered suture body 12.

Figure 7 schematically displays a surgical suture 10 comprising a suture body 12, an adhesive layer 14 and a flock material 16 and 18. The flock material 16 is unidirectionally disposed on the surface of the suture body 12 and adhesive layer 14, respectively. The flock material 18 which has a larger height than flock material 16 is annularly disposed at one end of the adhesive layered suture body 12, thereby forming a stopper element for the surgeon. Such a stopper element improves the handling of suture 10 inasmuch as it indicates to the surgeon when suture 10 is pulled ideally through tissue parts to be connected.

Figure 8 schematically displays a lateral view of a surgical suture 10 comprising an adhesive layered suture body 12 and a flock material 16 being attached to the adhesive layer of the suture body 12. The flock material 16 is unidirectionally disposed on the adhesive layered suture body 12. Further, the suture 10 comprises a loop portion 19 at one end of the suture body 12. The loop portion 19 is preferably free of flock material 16. In order to facilitate placement of the suture 10, the suture body 12 is typically attached to a needle 21 at its opposite end. The suture 10 may be advantageously employed as a knotless or self-fixating suture.

Preferably, the suture 10 comprises a core-sheath structure, wherein the core is formed by the suture body 12 and the sheath is formed by the adhesive layer. Further, the core may be made of polydioxanone, while the sheath may be made of polyhydroxybutyrate, particularly poly-4-hydroxybutyrate. The flock material 16 is preferably made of polydioxanone.

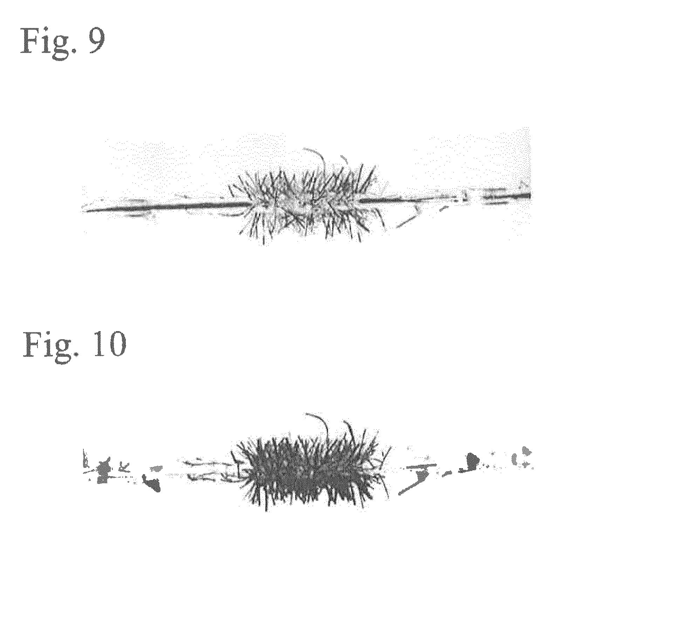

Figure 9 schematically shows a surgical suture comprising a suture body made of polydioxanone having a portion which is flocked with nylon fibres.

Figure 10 schematically shows a surgical suture comprising a suture body made of polypropylene having a portion which is flocked with nylon fibres.

### Examples

### Example 1: Manufacture of an absorbable thread comprising absorbable flock material

A monofilament having a core-sheath-structure, wherein the core was made of a terpolymer made of glycolide, trimethylene carbonate and ε-caprolactone and the sheath was made of polydioxanone, was passed through an oven with a speed of 5 m/min. The oven was heated to a temperature of 86°C, thereby allowing the sheath to be molten.

Immediately upon melting the sheath, the monofilament was transferred into a flocking cabin where the monofilament was flocked with fibres made of polyglycolide having a length of 0.3 mm. During the flocking process, the monofilament was continuously rotated.

For the flocking process, the monofilament was applied as an earthed electrode, along with a high-voltage electrode. The monofilament and the high-voltage electrode had a mutual distance of 40 cm. By means of a high-voltage generator (electrostat) a voltage of 60 kV was applied.

Due to the high-voltage, an electric field was generated between the monofilament to be flocked and the high-voltage electrode.

The polyglycolide fibres were provided by means of a dosing apparatus which was situated in the flocking cabin.

Upon passing the high-voltage electrode, the fibres adapted charges due to electrostatic induction and were accelerated along the streamlines of the electric field in the direction of the monofilament, where the fibres stuck to the molten sheath. Surplus polyglycolide fibres were continuously sucked off during the flocking process.

A subsequent drying step yielded a monofilament having a core-sheath-structure being flocked with fibres made of polyglycolide. Due to rotation during the flocking process, the monofilament was densely and circumferentially covered with polyglycolide fibres.

### Example 2: Manufacture of an absorbable thread comprising absorbable flock material

A monofilament having a core-sheath-structure, wherein the core was made of polydioxanone and the sheath was made of DL-lactide having a weight ratio of D-lactide to L-lactide of 7 : 3, was passed through an infrared chamber in a speed of 15 cm/s. The infrared chamber was heated to a temperature of approximately 90°C. The heat within the chamber led to the formation of a sticky sheath which acted as an adhesive layer in the following flocking process.

Directly behind the infrared chamber, a flocking cabin was situated to which the monofilament was conveyed after the sheath had been transferred into a sticky condition.

In the flocking cabin, the monofilament was flocked with fibres made of poly-4-hydroxybutyrate having a length of 1.0 mm. During the flocking process, the monofilament was continuously rotated.

For the flocking process, the monofilament was applied as an earthed electrode, along with a high-voltage electrode. The monofilament and the high-voltage electrode had a mutual distance of 30 cm. By means of a high-voltage generator (electrostat) a voltage of 60 kV was applied.

Due to the high-voltage, an electric field was generated between the monofilament to be flocked and the high-voltage electrode.

The poly-4-hydroxybutyrate fibres were provided by means of a dosing apparatus which was situated in the flocking cabin.

Upon passing the high-voltage electrode, the fibres adapted charges due to electrostatic induction and were accelerated along the streamlines of the electric field in the direction of the monofilament, where the fibres bond to the sticky sheath. Surplus poly-4-hydroxybutyrate fibres were continuously withdrawn by suction during the flocking process.

A subsequent drying step yielded a monofilament having a core-sheath-structure being flocked with fibres made of poly-4-hydroxybutyrate. Due to rotation during the flocking process, the monofilament was densely covered with poly-4-hydroxybutyrate fibres.

### Example 3: Manufacture of a non-absorbable braided thread comprising non-absorbable flock material

A braided polyester thread (Dacrofil USP1) was passed by means of rollers through a bath of a polyurethane based hot melt adhesive.

Afterwards, the coated thread was immediately transferred into a flocking cabin where the thread was flocked with fibres made of polyamide 6-6 having a length of 0.3 mm.

For the flocking process, the braided thread was applied as an earthed electrode, along with a high-voltage electrode. The thread and the high-voltage electrode had a mutual distance of 20 cm. By means of a high-voltage generator (electrostat) a voltage of 60 kV was applied.

Due to the high-voltage, an electric field was generated between the thread to be flocked and the high-voltage electrode.

The polyamide 6-6 fibres were provided by means of a dosing apparatus which was situated in the flocking cabin.

Upon passing the high-voltage electrode, the fibres adapted charges due to electrostatic induction and were accelerated along the streamlines of the electric field in the direction of the thread, where the fibres bound to the wet hot melt adhesive. Surplus polyamide 6/6 fibres were continuously sucked off during the flocking process.

A subsequent drying step yielded a braided thread being flocked with fibres made of polyamide 6-6.

### Example 4: Manufacture of an absorbable braided thread comprising helically arranged absorbable flock material

A braided thread made of polyglycolic acid having a helically run thread of lower melting poly-DL-lactide (weight ratio of D-lactide to L-lactide of 7 : 3) onto the exterior surface was passed through an oven (5 m) with the speed of 13 m/min. The oven was heated to an average temperature which was sufficient to melt the poly-DL-lactide thread.

After passing the oven, the thread was immediately transferred into a flocking cabin where the thread was flocked with poly-4-hydroxybutyrate fibres having a length of 0.8 mm. During the flocking process, the thread was continuously rotated.

For the flocking process, the thread was applied as an earthed electrode, along with a high-voltage electrode. The thread and the high-voltage electrode had a mutual distance of 30 cm. By means of a high-voltage generator (electrostat) a voltage of 60 kV was applied.

Due to the high-voltage, an electric field was generated between the thread to be flocked and the high-voltage electrode.

The poly-4-hydroxybutyrate fibres were provided by means of a dosing apparatus which was situated in the flocking cabin.

Upon passing the high-voltage electrode, the fibres adapted charges due to electrostatic induction and were accelerated along the streamlines of the electric field in the direction of the thread, where the fibres got stick to the molten poly-DL-lactide thread. Surplus poly-4-hydroxybutyrate fibres were continuously sucked off during the flocking process. A subsequent drying step yielded a thread being helically patterned with poly-4-hydroxybutyrate fibres. Due to rotation during the flocking process, the thread was densely covered with poly-4-hydroxybutyrate fibres.

### Example 5: Manufacture of an absorbable braid comprising absorbable flock material

A braid having a core-sheath structure was provided. The core of the braid was composed of 6 threads, while the sheath of the braid was composed of 16 threads. Each thread was in turn composed of 60 filaments embodying the typical braiding structure of a USP1 (Safil^{®}).

The core was completely made of polyglycolic acid. As regards the sheath, 4 threads were made of lower-melting polydioxanone, while the remaining 12 threads were made of polyglycolic acid.

Such a braid was flocked with poly-4-hydroxybutyrate as specified in example 4, thereby yielding a braid which was alternatively covered with poly-4-hydroxybutyrate fibres along the superficial course of the polydioxanone filaments.

### Example 6: Liquid Test

The middle finger of two natural latex gloves (Vasco sensitive powder free L/8-9 B. Braun) was punctured with a straight GS 60 needle (2 cm above the end of the finger).

The opening of one glove was stitched by a flocked suture as manufactured according to example 1. The opening of the other glove was stitched by means of an unflocked monofilament having a core-sheath structure, wherein the core was made of a terpolymer made of glycolide, trimethylencarbonate and ε-caprolactone and the sheath was made of polydioxanone.

Both gloves were filled with 500 ml deionised water and vertically hanged over a measuring flask. The released water volume was compared.

While the glove which was stitched by means of the unflocked monofilament had a water loss of 22.5 ml per hour, the glove which was stitched by the flocked suture merely exhibited a water loss of 4.0 ml per hour.

This result confirms that flocked sutures are useful for closing leakages, especially in suture stitch bleedings. This is in particular due to the voluminous structure of a flocked suture imparted by the flock material.

## Claims

1. Surgical suture comprising a suture body and a flock material, **characterized in that** the flock material is attached to the suture body by means of an adhesive layer.

2. Surgical suture according to claim 1, **characterized in that** the suture comprises a core-sheath structure, wherein the core is preferably formed by the suture body and the sheath is preferably formed by the adhesive layer.

3. Surgical suture according to claim 1 or 2, **characterized in that** the adhesive layer is made of a material, particularly a polymer, having a lower melting temperature than a material, particularly a polymer, of which the suture body is made.

4. Surgical suture according to any of the preceding claims, **characterized in that** the adhesive layer includes or is made of a material selected from the group consisting of hot melts, polyvinyl alcohol, polyvinyl acetate, polyvinylchloride, polyvinyl propionate, polyacrylates, cyanoacrylates, epoxy resins, acrylic resins, resin sizes such as urea resin size, polyurethanes, polyhydroxyalkanoates, proteins such as collagen, gelatine, elastin, fibronectin and the like, polysaccharides, in particular mucopolysaccharides, copolymers thereof, stereoisomers thereof, salts thereof, and combinations thereof.

5. Surgical suture according to any of the preceding claims, **characterized in that** the flock material is formed as fibres, in particular as milled or crushed fibres.

6. Surgical suture according to any of the preceding claims, **characterized in that** the flock material is arranged in a pattern-like disposition onto the suture body, in particular onto the adhesive layer, preferably selected from the group consisting of linear disposition, staggered disposition, helical or spiral disposition, meander-like disposition, serpentine-like disposition, random or irregular disposition, annular disposition, and combinations thereof.

7. Surgical suture according to any of the preceding claims, **characterized in that** the flock material includes or is made of a material which is selected from the group consisting of polyolefins, polyvinyl alcohols, polyamides, polyimides, polyesters, polyurethanes, in particular thermoplastic polyurethanes, polyhydroxyalkanoates, proteins, polysaccharides, copolymers thereof, stereoisomers thereof, salts thereof, and combinations thereof, wherein the flock material more preferably includes or is made of a material which is selected from the group consisting of polyethylene, low-density polyethylene, high-density polyethylene, high-molecular-weight polyethylene, ultra-high-molecular-weight polyethylene, polypropylene, polyethylene terephthalate, polypropylene terephthalate, polybutylene terephthalate, polyamide 6, polyamide 6-6, polyamide 6-12, polyamide 12, polytetrafluorethylene, polyvinylidene dichloride, polyvinylidene difluoride, polytetrafluorpropylene, polyhexafluorpropylene, polyvinylalcohol, polyglycolide, polylactide, polydioxanone, polyhydroxybutyrate, poly-3-hydroxybutyrate, poly-4-hydroxybutyrate, polytrimethylenecarbonate, poly-ε-caprolactone, collagen, gelatine, elastin, reticulin, fibronectin, laminin, fibrin, fibrinogen, albumin, starch, amylose, amylopectin, dextran, cellulose, methycellulose, carboxymethylcellulose, chitosan, hyaluronic acid, dextran sulfate, heparin, heparan sulfate, chondroitin sulfate, dermatan sulfate, copolymers thereof, stereoisomers thereof, salts thereof, and combinations thereof.

8. Surgical suture according to any of the claims 1 to 6, **characterized in that** the flock material includes or is made of an active agent, in particular a cosmetic, pharmaceutical and/or medicinal agent, preferably selected from the group consisting of antibiotics, disinfectants, oncological agents, anti-scarring agents such as captopril, inflammation inhibitors, pain-killing agents, growth factors, cellular differentiating factors, cellular adhesion factors, cellular recruiting factors, cell receptors, cell binding factors, cytokines, peptides, proteins such as a collagen, lipids, polysaccharides such as hyaluronic acids, oligonucleotides, polynucleotides, DNA, RNA, stereosiomers thereof, salts thereof, and combinations thereof.

9. Surgical suture according to any of the preceding claims, **characterized in that** the surgical suture is a monofile suture, a pseudo monofile suture, a textured suture or a multifile suture, in particular an intertwined suture, a twisted suture or a braided suture.

10. Surgical suture according to any of the preceding claims, **characterized in that** the suture body includes or is made of a material which is selected from the group consisting of polyolefins, polyvinyl alcohols, polyamides, polyimides, polyesters, polyurethanes, in particular thermoplastic polyurethanes, polyhydroxyalkanoates, proteins, polysaccharides, copolymers thereof, stereoisomers thereof, salts thereof, and combinations thereof, wherein the suture body more preferably includes or is made of a material which is selected from the group consisting of polyethylene, low-density polyethylene, high-density polyethylene, high-molecular-weight polyethylene, ultra-high-molecular-weight polyethylene, polypropylene, polyethylene terephthalate, polypropylene terephthalate, polybutylene terephthalate, polyamide 6, polyamide 6-6, polyamide 6-12, polyamide 12, polytetrafluorethylene, polyvinylidene dichloride, polyvinylidene difluoride, polytetrafluorpropylene, polyhexafluorpropylene, polyvinyl alcohol, polyglycolide, polylactide, polydioxanone, polyhydroxybutyrate, poly-3-hydroxybutyrate, poly-4-hydroxybutyrate, polytrimethylene carbonate, poly-s-caprolactone, collagen, gelatine, elastin, reticulin, fibronectin, laminin, fibrin, albumin, starch, amylose, amylopectin, dextran, cellulose, methycellulose, carboxymethylcellulose, chitosan, hyaluronic acid, dextran sulfate, heparin, heparan sulfate, chondroitin sulfate, dermatan sulfate, copolymers thereof, stereoisomers thereof, salts thereof, and combinations thereof.

11. Surgical suture according to any of the preceding claims, **characterized in that** the suture is a self-fixating suture.

12. Surgical suture according to any of the preceding claims, **characterized in that** the suture body comprises a unidirectionally orientated flock material and a terminally positioned stopper element, wherein the stopper element is preferably made of annularly disposed flock material having a larger height than the unidirectionally orientated flock material.

13. Method of manufacturing a surgical suture, in particular according to any of the preceding claims, comprising the steps of
a) layering a suture body with an adhesive,
b) flocking the adhesive-layered suture body with a flock material while the adhesive is still active, in particular wet, or upon activation of the adhesive.

14. Method of manufacturing a surgical suture, in particular according to any of the claims 1 to 12, comprising the steps of
a) providing a suture body which is at least partly made of a material which acts as an adhesive upon activation,
b) flocking the suture body with a flock material upon activation of the suture body's material.

15. Surgical combination, in particular surgical kit, comprising a surgical suture according to any of the claims 1 to 12, and at least one surgical needle.

## Patentansprüche

1. Chirurgisches Nahtmaterial umfassend einen Nahtmaterialkörper und ein Flockmaterial, **dadurch gekennzeichnet, dass** das Flockmaterial am Nahtmaterialkörper mittels einer Klebstoffschicht angebracht ist.

2. Chirurgisches Nahtmaterial nach Anspruch 1, **dadurch gekennzeichnet, dass** das Nahtmaterial eine Kern-Mantel-Struktur umfasst, wobei der Kern bevorzugt durch den Nahtmaterialkörper gebildet ist und der Mantel bevorzugt durch die Klebstoffschicht gebildet ist.

3. Chirurgisches Nahtmaterial nach Anspruch 1 oder 2, **dadurch gekennzeichnet, dass** die Klebstoffschicht aus einem Material, insbesondere einem Polymer, gebildet ist, das eine niedrigere Schmelztemperatur aufweist als ein Material, insbesondere ein Polymer, aus dem der Nahtmaterialkörper gebildet ist.

4. Chirurgisches Nahtmaterial nach einem der vorhergehenden Ansprüche, **dadurch gekennzeichnet, dass** die Klebstoffschicht ein Material beinhaltet oder daraus gebildet ist, das ausgewählt ist aus der Gruppe bestehend aus Schmelzklebstoffen, Polyvinylalkohol, Polyvinylacetat, Polyvinylchlorid, Polyvinylpropionat, Polyacrylaten, Cyanoacrylaten, Epoxidharzen, Acrylharzen, Harzleimen wie Harnstoffharzleim, Polyurethanen, Polyhydroxyalkanoaten, Proteinen wie Kollagen, Gelatine, Elastin, Fibronectin und dergleichen, Polysacchariden, insbesondere Mucopolysacchariden, Copolymeren davon, Stereoisomeren davon, Salzen davon und Kombinationen davon.

5. Chirurgisches Nahtmaterial nach einem der vorhergehenden Ansprüche, **dadurch gekennzeichnet, dass** das Flockmaterial als Fasern, insbesondere als gemahlene oder zerkleinerte Fasern, ausgebildet ist.

6. Chirurgisches Nahtmaterial nach einem der vorhergehenden Ansprüche, **dadurch gekennzeichnet, dass** das Flockmaterial in einer musterartigen Anordnung auf dem Nahtmaterialkörper, insbesondere auf der Klebstoffschicht, vorgesehen ist, bevorzugt ausgewählt aus der Gruppe bestehend aus einer linearen Anordnung, versetzten Anordnung, schneckenförmigen oder spiralförmigen Anordnung, mäanderförmigen Anordnung, serpentinenartigen Anordnung, statistischer oder unregelmäßiger Anordnung, ringförmiger Anordnung und Kombinationen davon.

7. Chirurgisches Nahtmaterial nach einem der vorhergehenden Ansprüche, **dadurch gekennzeichnet, dass** das Flockmaterial ein Material beinhaltet oder daraus gebildet ist, das ausgewählt ist aus der Gruppe bestehend aus Polyolefinen, Polyvinylalkoholen, Polyamiden, Polyimiden, Polyestern, Polyurethanen, insbesondere thermoplastischen Polyurethanen, Polyhydroxyalkanoaten, Proteinen, Polysacchariden, Copolymeren davon, Stereoisomeren davon, Salzen davon und Kombinationen davon, wobei das Flockmaterial besonders bevorzugt ein Material beinhaltet oder daraus gebildet ist, das ausgewählt ist aus der Gruppe bestehend aus Polyethylen, Polyethylen niedriger Dichte, Polyethylen hoher Dichte, hochmolekularem Polyethylen, ultrahochmolekularem Polyethylen, Polypropylen, Polyethylenterephthalat, Polypropylenterephthalat, Polybutylenterephthalat, Polyamid 6, Polyamid 6-6, Polyamid 6-12, Polyamid 12, Polytetrafluorethylen, Polyvinylidendichlorid, Polyvinylidendifluorid, Polytetrafluorpropylen, Polyhexafluorpropylen, Polyvinylalkohol, Polyglycolid, Polylactid, Polydioxanon, Polyhydroxybutyrat, Poly-3-hydroxybutyrat, Poly-4-hydroxybutyrat, Polytrimethylencarbonat, Poly-ε-caprolacton, Kollagen, Gelatine, Elastin, Reticulin, Fibronectin, Laminin, Fibrin, Fibrinogen, Albumin, Stärke, Amylose, Amylopectin, Dextran, Cellulose, Methylcellulose, Carboxymethylcellulose, Chitosan, Hyaluronsäure, Dextransulfat, Heparin, Heparansulfat, Chondroitinsulfat, Dermatansulfat, Copolymeren davon, Stereoisomeren davon, Salzen davon und Kombinationen davon.

8. Chirurgisches Nahtmaterial nach einem der Ansprüche 1 bis 6, **dadurch gekennzeichnet, dass** das Flockmaterial einen Wirkstoff, insbesondere einen kosmetischen, pharmazeutischen und/oder medizinischen Wirkstoff, beinhaltet oder daraus gebildet ist, der bevorzugt ausgewählt ist aus der Gruppe bestehend aus Antibiotika, Desinfektionsmitteln, onkologischen Wirkstoffen, Antinarbenmitteln wie Captopril, Entzündungshemmern, Schmerzmitteln, Wachstumsfaktoren, Zelldifferenzierungsfaktoren, Zelladhäsionsfaktoren, Zellrekrutierungsfaktoren, Zellrezeptoren, Zellbindungsfaktoren, Cytokinen, Peptiden, Proteinen wie Kollagen, Lipiden, Polysacchariden, wie Hyaluronsäuren, Oligonukleotiden, Polynukleotiden, DNA, RNA, Stereosisomeren davon, Salzen davon und Kombinationen davon.

9. Chirurgisches Nahtmaterial nach einem der vorhergehenden Ansprüche, **dadurch gekennzeichnet, dass** das chirurgische Nahtmaterial als monofiles Nahtmaterial, pseudomonofiles Nahtmaterial, texturiertes Nahtmaterial oder multifiles Nahtmaterial, insbesondere als verschränktes Nahtmaterial, verzwirntes Nahtmaterial oder geflochtenes Nahtmaterial, ausgebildet ist.

10. Chirurgisches Nahtmaterial nach einem der vorhergehenden Ansprüche, **dadurch gekennzeichnet, dass** der Nahtmaterialkörper ein Material beinhaltet oder daraus gebildet ist, das ausgewählt ist aus der Gruppe bestehend aus Polyolefinen, Polyvinylalkoholen, Polyamiden, Polyimiden, Polyestern, Polyurethanen, insbesondere thermoplastischen Polyurethanen, Polyhydroxyalkanoaten, Proteinen, Polysacchariden, Copolymeren davon, Stereoisomeren davon, Salzen davon und Kombinationen davon, wobei der Nahtmaterialkörper besonders bevorzugt ein Material beinhaltet oder daraus gebildet ist, das ausgewählt ist aus der Gruppe bestehend aus Polyethylen, Polyethylen niedriger Dichte, Polyethylen hoher Dichte, hochmolekularem Polyethylen, ultrahochmolekularem Polyethylen, Polypropylen, Polyethylenterephthalat, Polypropylenterephthalat, Polybutylenterephthalat, Polyamid 6, Polyamid 6-6, Polyamid 6-12, Polyamid 12, Polytetrafluorethylen, Polyvinylidendichlorid, Polyvinylidendifluorid, Polytetrafluorpropylen, Polyhexafluorpropylen, Polyvinylalkohol, Polyglycolid, Polylactid, Polydioxanon, Polyhydroxybutyrat, Poly-3-hydroxybutyrat, Poly-4-hydroxybutyrat, Polytrimethylencarbonat, Poly-ε-caprolacton, Kollagen, Gelatine, Elastin, Reticulin, Fibronectin, Laminin, Fibrin, Albumin, Stärke, Amylose, Amylopectin, Dextran, Cellulose, Methylcellulose, Carboxymethylcellulose, Chitosan, Hyaluronsäure, Dextransulfat, Heparin, Heparansulfat, Chondroitinsulfat, Dermatansulfat, Copolymeren davon, Stereoisomeren davon, Salzen davon und Kombinationen davon.

11. Chirurgisches Nahtmaterial nach einem der vorhergehenden Ansprüche, **dadurch gekennzeichnet, dass** das chirurgische Nahtmaterial als selbstfixierendes Nahtmaterial ausgebildet ist.

12. Chirurgisches Nahtmaterial nach einem der vorhergehenden Ansprüche, **dadurch gekennzeichnet, dass** der Nahtmaterialkörper ein unidirektional orientiertes Flockmaterial und ein endständig positioniertes Stopperelement umfasst, wobei das Stopperelement bevorzugt aus ringförmig angeordnetem Flockmaterial mit einer größeren Höhe als das unidirektional orientierte Flockmaterial gebildet ist.

13. Verfahren zur Herstellung eines chirurgischen Nahtmaterials, insbesondere nach einem der vorhergehenden Ansprüche, umfassend die Schritte
a) Beschichten eines Nahtmaterialkörpers mit einem Klebstoff,
b) Beflocken des klebstoffbeschichteten Nahtmaterialkörpers mit einem Flockmaterial, während der Klebstoff noch aktiv, insbesondere nass, ist oder nach Aktivierung des Klebstoffs.

14. Verfahren zur Herstellung eines chirurgischen Nahtmaterials, insbesondere nach einem der Ansprüche 1 bis 12, umfassend die Schritte
a) Bereitstellen eines Nahtmaterialkörpers, der mindestens teilweise aus einem Material gebildet ist, das nach Aktivierung als Klebstoff wirkt,
b) Beflocken des Nahtmaterialkörpers mit einem Flockmaterial nach Aktivierung des Materials des Nahtmaterialkörpers.

15. Chirurgische Kombination, insbesondere chirurgisches Kit, umfassend ein chirurgisches Nahtmaterial nach einem der Ansprüche 1 bis 12 und mindestens eine chirurgische Nadel.

## Revendications

1. Suture chirurgicale comprenant un corps de suture et un matériau de flocage, **caractérisée en ce que** le matériau de flocage est fixé au corps de suture au moyen d'une couche d'adhésif.

2. Suture chirurgicale selon la revendication 1, **caractérisée en ce que** la suture comprend une structure noyau-enveloppe, le noyau étant préférablement formé du corps de suture et l'enveloppe étant préférablement formée de la couche d'adhésif.

3. Suture chirurgicale selon la revendication 1 ou 2, **caractérisée en ce que** la couche d'adhésif se compose d'un matériau, en particulier un polymère, ayant une température de fusion inférieure à celle d'un matériau, en particulier un polymère, dont se compose le corps de suture.

4. Suture chirurgicale selon l'une quelconque des revendications précédentes, **caractérisée en ce que** la couche d'adhésif contient ou se compose d'un matériau sélectionné dans le groupe constitué des matériaux thermofusibles, de l'alcool polyvinylique, de l'acétate de polyvinyle, du chlorure de polyvinyle, du propionate de polyvinyle, des polyacrylates, des cyanoacrylates, des résines époxydes, des résines acryliques, des encollages de résine tels qu'un encollage de résine d'urée, des polyuréthanes, des polyhydroxyalcanoates, de protéines telles que le collagène, la gélatine, l'élastine, la fibronectine et des matériaux similaires, des polysaccharides, en particulier les mucopolysaccharides, de copolymères de ceux-ci, de stéréoisomères de ceux-ci, de sels de ceux-ci, et de combinaisons de ceux-ci.

5. Suture chirurgicale selon l'une quelconque des revendications précédentes, **caractérisée en ce que** le matériau de flocage est sous forme de fibres, en particulier sous forme de fibres écrasées ou broyées.

6. Suture chirurgicale selon l'une quelconque des revendications précédentes, **caractérisée en ce que** le matériau de flocage est arrangé selon une disposition similaire à un motif sur le corps de suture, en particulier sur la couche d'adhésif, préférablement sélectionnée dans le groupe constitué d'une disposition linéaire, d'une disposition décalée, d'une disposition hélicoïdale ou en spirale, d'une disposition sinueuse, d'une disposition onduleuse, d'une disposition aléatoire ou irrégulière, d'une disposition annulaire, et de combinaisons de celles-ci.

7. Suture chirurgicale selon l'une quelconque des revendications précédentes, **caractérisée en ce que** le matériau de flocage contient ou se compose d'un matériau qui est sélectionné dans le groupe constitué des polyoléfines, des alcools polyvinyliques, des polyamides, des polyimides, des polyesters, des polyuréthanes, en particulier des polyuréthanes thermoplastiques, des polyhydroxyalcanoates, des protéines, des polysaccharides, de copolymères de ceux-ci, de stéréoisomères de ceux-ci, de sels de ceux-ci, et de combinaisons de ceux-ci, le matériau de flocage contenant ou se composant plus préférablement d'un matériau qui est sélectionné dans le groupe constitué du polyéthylène, du polyéthylène de faible densité, du polyéthylène de haute densité, du polyéthylène à haute masse moléculaire, du polyéthylène à ultra-haute masse moléculaire, du polypropylène, du téréphtalate de polyéthylène, du téréphtalate de polypropylène, du téréphtalate de polybutylène, du polyamide 6, du polyamide 6-6, du polyamide 6-12, du polyamide 12, du polytétrafluoroéthylène, du dichlorure de polyvinylidène, du difluorure de polyvinylidène, du polytétrafluoropropylène, du polyhexafluoropropylène, de l'alcool polyvinylique, du polyglycolide, du polylactide, de la polydioxanone, du polyhydroxybutyrate, du poly-3-hydroxybutyrate, du poly-4-hydroxybutyrate, du carbonate de polytriméthylène, de la poly-ε-caprolactone, du collagène, de la gélatine, de l'élastine, de la réticuline, de la fibronectine, de la laminine, la fibrine, du fibrinogène, de l'albumine, de l'amidon, de l'amylose, de l'amylopectine, du dextrane, de la cellulose, de la méthylcellulose, de la carboxyméthylcellulose, du chitosane, de l'acide hyaluronique, du sulfate de dextrane, de l'héparine, du sulfate d'héparane, du sulfate de chondroïtine, du sulfate de dermatane, de copolymères de ceux-ci, de stéréoisomères de ceux-ci, de sels de ceux-ci, et de combinaisons de ceux-ci.

8. Suture chirurgicale selon l'une quelconque des revendications 1 à 6, **caractérisée en ce que** le matériau de flocage contient ou se compose d'un agent actif, en particulier un agent cosmétique, pharmaceutique et/ou médicinal, préférablement sélectionné dans le groupe constitué des antibiotiques, des désinfectants, des agents oncologiques, des agents inhibant la formation de cicatrices tels que le captopril, des anti-inflammatoires, des analgésiques, des facteurs de croissance, des facteurs de différenciation cellulaire, des facteurs d'adhésion cellulaire, des facteurs de recrutement cellulaire, des récepteurs cellulaires, des facteurs de liaisons cellulaires, des cytokines, des peptides, de protéines telles qu'un collagène, des lipides, de polysaccharides tels que les acides hyaluroniques, des oligonucléotides, des polynucléotides, de l'ADN, de l'ARN, de stéréoisomères de ceux-ci, de sels de ceux-ci, et de combinaisons de ceux-ci.

9. Suture chirurgicale selon l'une quelconque des revendications précédentes, **caractérisée en ce que** la suture chirurgicale est une suture monofil, une suture pseudo-monofil, une suture texturée ou une suture multifil, en particulier une suture entrelacée, une suture torsadée ou une suture tressée.

10. Suture chirurgicale selon l'une quelconque des revendications précédentes, **caractérisée en ce que** le corps de suture contient ou se compose d'un matériau qui est sélectionné dans le groupe constitué des polyoléfines, des alcools polyvinyliques, des polyamides, des polyimides, des polyesters, des polyuréthanes, en particulier des polyuréthanes thermoplastiques, des polyhydroxyalcanoates, des protéines, des polysaccharides, de copolymères de ceux-ci, de stéréoisomères de ceux-ci, de sels de ceux-ci, et de combinaisons de ceux-ci, le corps de suture contenant ou se composant plus préférablement d'un matériau qui est sélectionné dans le groupe constitué du polyéthylène, du polyéthylène de faible densité, du polyéthylène de haute densité, du polyéthylène à haute masse moléculaire, du polyéthylène à ultra-haute masse moléculaire, du polypropylène, du téréphtalate de polyéthylène, du téréphtalate de polypropylène, du téréphtalate de polybutylène, du polyamide 6, du polyamide 6-6, du polyamide 6-12, du polyamide 12, du polytétrafluoroéthylène, du dichlorure de polyvinylidène, du difluorure de polyvinylidène, du polytétrafluoropropylène, du polyhexafluoropropylène, de l'alcool polyvinylique, du polyglycolide, du polylactide, de la polydioxanone, du polyhydroxybutyrate, du poly-3-hydroxybutyrate, du poly-4-hydroxybutyrate, du carbonate de polytriméthylène, de la poly-ε-caprolactone, du collagène, de la gélatine, de l'élastine, de la réticuline, de la fibronectine, de la laminine, la fibrine, de l'albumine, de l'amidon, de l'amylose, de l'amylopectine, du dextrane, de la cellulose, de la méthylcellulose, de la carboxyméthylcellulose, du chitosane, de l'acide hyaluronique, du sulfate de dextrane, de l'héparine, du sulfate d'héparane, du sulfate de chondroïtine, du sulfate de dermatane, de copolymères de ceux-ci, de stéréoisomères de ceux-ci, de sels de ceux-ci, et de combinaisons de ceux-ci.

11. Suture chirurgicale selon l'une quelconque des revendications précédentes, **caractérisée en ce que** la suture est une suture auto-fixante.

12. Suture chirurgicale selon l'une quelconque des revendications précédentes, **caractérisée en ce que** le corps de suture comprend un matériau de flocage à orientation unidirectionnelle et un élément de butée occupant une position terminale, l'élément de butée étant préférablement constitué de matériau de flocage à disposition annulaire ayant une hauteur supérieure à celle du matériau de flocage à orientation unidirectionnelle.

13. Procédé de fabrication d'une suture chirurgicale, en particulier selon l'une quelconque des revendications précédentes, comprenant les étapes qui consistent à :
a) revêtir un corps de suture d'une couche d'adhésif,
b) floquer le corps de suture revêtu d'une couche d'adhésif avec un matériau de flocage alors que l'adhésif est encore actif, en particulier mouillé, ou au moment de l'activation de l'adhésif.

14. Procédé de fabrication d'une suture chirurgicale, en particulier selon l'une quelconque des revendications 1 à 12, comprenant les étapes qui consistent à :
a) fournir un corps de suture qui est constitué au moins en partie d'un matériau qui agit comme un adhésif quand il est activé,
b) floquer le corps de suture avec un matériau de flocage au moment de l'activation du matériau du corps de suture.

15. Combinaison chirurgicale, en particulier trousse chirurgicale, comprenant une suture chirurgicale selon l'une quelconque des revendications 1 à 12, et au moins une aiguille chirurgicale.
